# EUROPEAN PATENT SPECIFICATION

(11) **EP 2 730 535 B1**
(45) Date of publication and mention of the grant of the patent: **29.06.2016**
(21) Application number: 12811815.5
(22) Date of filing: 03.04.2012
(51) Int. Cl.: B67D 1/07, A61L 9/015, A61L 9/20

(54) **WATER DISPENSER WITH OZONE AIR STERILIZATION CHAMBER**
WASSERSPENDER MIT OZON-LUFTSTERILISATIONSKAMMER
DISTRIBUTEUR D'EAU AVEC STÉRILISATEUR D'OZONE

(30) Priority: 08.07.2011 JP 2011151481
(43) Date of publication of application: 14.05.2014
(73) Proprietor: Kabushiki Kaisha Cosmo Life, Kakogawa-shi Hyogo 675-0032 (JP)
(72) Inventor: SHIOTANI Masaru, Fukuchiyama-shi Kyoto 620-0926 (JP); SHIOTANI Nana, Fukuchiyama-shi Kyoto 620-0926 (JP)
(74) Representative: Grünecker Patent- und Rechtsanwälte PartG mbB
(86) International application number: PCT/JP2012/059062
(87) International publication number: WO 2013/008496

(56) References cited:
- WO-A1-2006/015480
- WO-A1-2010/023770
- WO-A1-2013/045508
- JP-A- 3 001 024
- JP-A- 2006 341 915
- JP-A- 2007 136 415
- JP-A- 2010 052 752
- JP-A- 2011 010 835
- JP-B1- 4 317 259
- US-A- 5 366 619
- DATABASE WPI Week 200957 Thomson Scientific, London, GB; AN 2009-M75039 XP002727769, -& JP 4 317259 B1 (COSMO LIFE KK) 19 August 2009 (2009-08-19)

## Description

This invention relates to a water server through which drinking water, such as mineral water, in a water bottle can be dispensed.

Water servers are increasingly used e.g. in offices and hospitals these days. Ordinary water serves include a cold water tank which can store part of drinking water in a water bottle detachably mounted on the water server, a water cooling means for cooling the water in the cold water tank, and a water dispensing means for dispensing cold water in the cold water tank into e.g. a paper cup.

As water is dispensed from such a water server, the water level in the cold water tank falls, so that air is introduced into the cold water tank in an amount corresponding to the fall of the water level. If the air introduced into the cold water tank contains bacteria, the bacteria may be mixed into the drinking water in the cold water tank, which is not hygienically favorable. In order to keep the drinking water in the cold water tank hygienic, the inventor of the present application proposed a water server disclosed in the below-identified Patent document 1.

The water server disclosed in Patent document 1 includes a cold water tank in which drinking water can be stored and cooled, a cold water discharge pipe through which drinking water is dispensed from the cold water tank, and an air inlet pipe through which air is introduced into the cold water tank as the water level in the cold water tank falls. An air sterilizing chamber is connected to the air inlet pipe which sterilizes air in the pipe with ozone.

As shown in Fig. 5, the air sterilizing chamber 40 disclosed in Patent document 1 has a case 41 in which a plurality of air compartments 43 are defined by partitioning walls 42 so as to be arranged one over another. The air compartment 43 at the lowest level has an air inlet 44 through-outer air is introduced into the case 41. The air sterilizing chamber further includes an ozone generator 45 mounted in the air compartment 43 at the highest level and configured to convert oxygen in the air to ozone. The air compartment 43 at the highest level has an air outlet 46 through which air in the case 41 is discharged out of the case 41. The partitioning walls 43, separating the vertically adjacent air compartments 43, are inclined alternately in opposite directions. Minute air holes 47 are formed in the lower end portion 42a of each inclined partitioning wall 42 through which the vertically adjacent air compartments 43 communicate with each other. In order to prevent ozone in the case 41 from being released out of the case, activated charcoal filters 48 and 49 are provided at the air inlet 44 and the air outlet 46, respectively.

During use of the air sterilizing chamber 40, ozone generated by the ozone generator 45, which is mounted in the air compartment 43 at the highest level, descend in the case 41 because ozone is larger in specific gravity than air. On the other hand, air introduced into the case 41 through the air inlet 44 provided in the air compartment 43 at the lowest level rises in the case 41 and leaves the case 41 through the air outlet 46 provided in the air compartment 43 at the highest level. While rising in the case 41 through the air inlet 44, the air is brought into contact with the downwardly flowing ozone. Thus the air is sterilized and cleaned by the time it leaves the case 41.

### PRIOR ART DOCUMENT(S)

### PATENT DOCUMENT(S)

Patent document 1: JP Patent 4317259B

With the air sterilizing chamber 40 disclosed in Patent document 1, since the activated charcoal filter 48 is provided in the air compartment 43 at the lowest level, ozone generated by the ozone generator 45 is continuously brought into contact with and decomposed by this activated charcoal filter 48 when the ozone descends in the case 41 due to the difference in specific gravity between ozone and air. Thus, it is difficult to sufficiently increase the ozone concentration in the case 41, which could in turn make it difficult to sufficiently sterilize air that passes through the case 41.

Another problem with the air sterilizing chamber 40 disclosed in Patent document 1 is that since the activated charcoal filter 49 is provided in the air compartment 43 at the highest level, ozone in the air compartment 43 at the highest level tends to be brought into contact with and decomposed by this activated charcoal filter 49. Thus, while air in and out of the case 41 is stationary (while no drinking water is being dispensed from the water server), the ozone concentration tends to be especially low in the air compartment 43 at the highest level.

An object of the present invention is to provide a water server that effectively sterilize air to be brought into contact with drinking water in a water server.

According to the present invention said object is solved by a water server having the features of independent claim 1. Preferred embodiments are laid down in the dependent claims.

Accordingly, it is provided a water server comprising a water tank which can hold drinking water, a water discharge pipe through which drinking water in the water tank can be discharged, an air introducing pipe through which air can be introduced into the water tank as a water level in the water tank falls, and an air sterilizing chamber connected to the air introducing pipe and configured to sterilize air in the air introducing pipe, wherein the air sterilizing chamber comprises a case having an air inlet port and an air outlet port, and defining in the case an air compartment, an air inlet passage through which the air compartment communicate with the air inlet port, and an air outlet passage through which the air compartment communicates with the air outlet port, an ozone generator mounted in the air compartment and capable of converting oxygen in the air compartment to ozone, an inlet-side ozone-decomposing filter mounted in the air inlet passage at a higher level than the air compartment, and an outlet-side ozone-decomposing filter mounted in the air outlet passage at a higher level than the air compartment.

With this arrangement, since the inlet-side ozone-decomposing filter and the outlet-side ozone-decomposing filter are located at higher levels than the air compartment, when ozone generated by the ozone generator descends in the case due to the difference in specific gravity between ozone and air, the ozone is never brought into contact with either of the inlet-side ozone decomposing filter and the outlet-side ozone decomposing filter. Thus, ozone generated by the ozone generator almost entirely stays in the air compartment, so that it is possible to efficiently increase the ozone concentration in the case, and thus to effectively sterilize air that passes through the case.

Preferably, the air inlet passage includes a portion extending between the air compartment and the inlet-side ozone-decomposing filter and partially extending above the inlet-side ozone-decomposing filter, and the air outlet passage includes a portion extending between the air compartment and the outlet-side ozone-decomposing filter and partially extending above the outlet-side ozone-decomposing filter. With this arrangement, since the path between the air compartment and the inlet-side ozone decomposing filter has a portion extending at a higher level than the inlet-side ozone decomposing filter, ozone, which is larger in specific gravity than air, is even less likely to reach the inlet-side ozone decomposing filter. Similarly, since the path between the air compartment and the outlet-side ozone decomposing filter has a portion extending at a higher level than the outlet-side ozone decomposing filter, ozone, which is larger in specific gravity than air, is less likely to reach the outlet-side ozone decomposing filter. This makes it possible to prevent ozone in the air compartment from being brought into contact with either of the ozone-decomposing filters while air in and out of the case is stationary (while no drinking water is being dispensed from the water server), which in turn makes it possible to effectively increase the ozone concentration in the case.

Preferably, the air inlet passage includes a rectangular spiral path enclosing the inlet-side ozone-decomposing filter, and the air outlet passage includes a rectangular spiral path enclosing the outlet-side ozone-decomposing filter. Such rectangular spiral paths serve to increase the total lengths of the air inlet passage and the air outlet passage, provided the interior of the case remains unchanged. This arrangement thus further effectively prevents ozone in the air compartment from reaching, and being decomposed by, either of the inlet-side ozone-decomposing filter and the outlet-side ozone-decomposing filter.

The present **disclosure** also provides an air sterilizing chamber for use in a water server, comprising a case having an air inlet port and an air outlet port, and defining in the case an air compartment, an air inlet passage through which the air compartment communicate with the air inlet port, and an air outlet passage through which the air compartment communicates with the air outlet port, an ozone generator mounted in the air compartment and capable of converting oxygen in the air compartment to ozone, an inlet-side ozone-decomposing filter mounted in the air inlet passage at a higher level than the air compartment, and an outlet-side ozone-decomposing filter mounted in the air outlet passage at a higher level than the air compartment.

### ADVANTAGES OF THE INVENTION

With the water server according to the present invention, since the inlet-side ozone decomposing filter and the outlet-side ozone decomposing filter are provided in the air sterilizing chamber at higher levels than the air compartment, ozone generated by the ozone generator is less likely to be brought into contact with either of the inlet-side ozone decomposing filter and the outlet-side ozone decomposing filter. This makes it possible to efficiently increase the ozone concentration in the case of the air sterilizing chamber and thus to effectively sterilize air to be brought into contact with drinking water in the water tank.

### BRIEF DESCRIPTION OF THE DRAWINGS

Fig. 1 is a sectional view of a water server embodying the present invention.
Fig. 2 is a sectional view taken along line II-II of Fig. 1.
Fig. 3 is a sectional view taken along line III-III of Fig. 2.
Fig. 4 is a partial sectional view of a different water server.

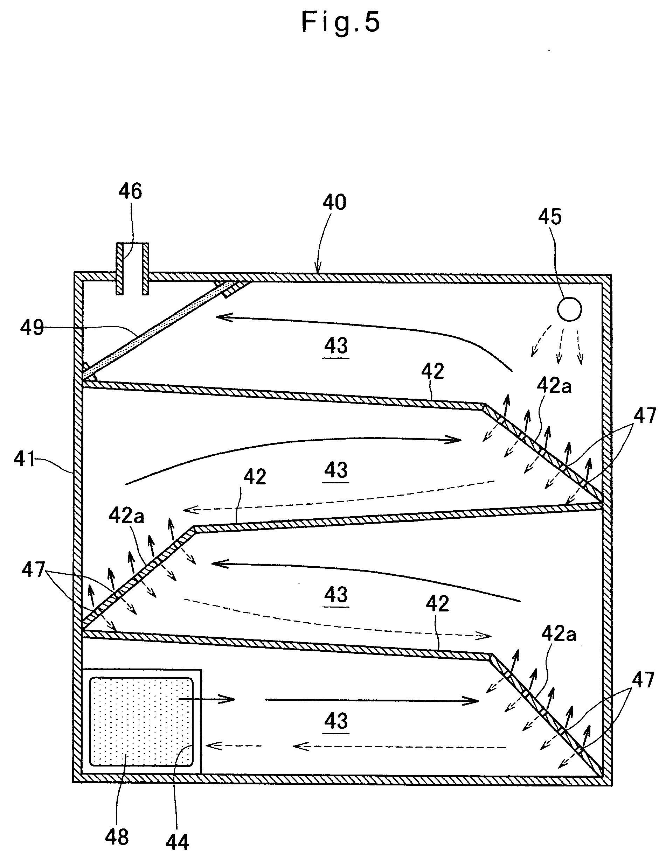

Fig. 5 is a sectional view of a conventional air sterilizing chamber for use in a water server.

### BEST MODE FOR EMBODYING THE INVENTION

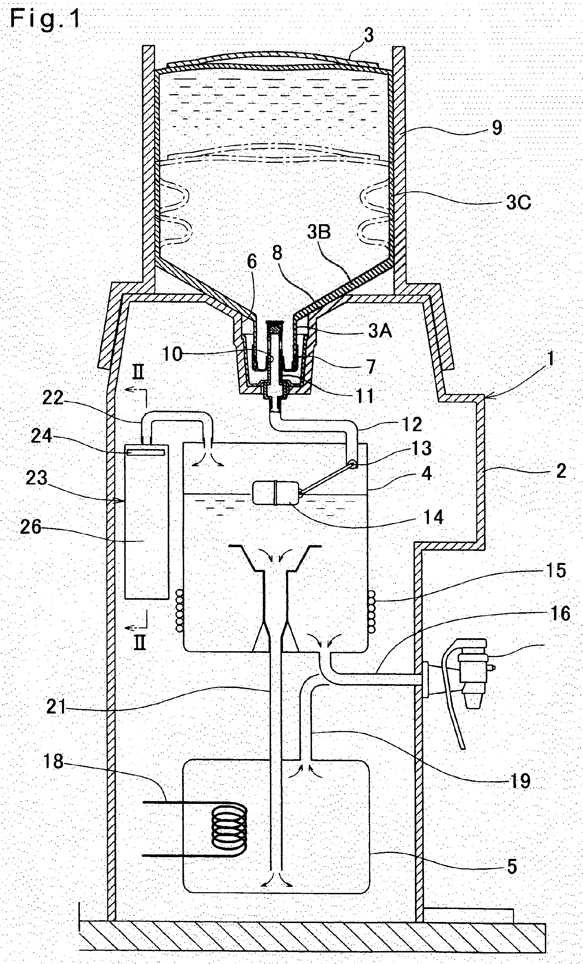

Fig. 1 shows the water server 1 embodying the present invention. The water server 1 includes a housing 2, a cold water tank 4 mounted in the housing 2 and configured to receive and cool a portion of drinking water in a water bottle 3 detachably set on top of the housing 2, and a hot water tank 5 mounted in the housing 2 under the cold water tank 4.

The water bottle 3 comprises a neck 3A which can be inserted in a bottle insertion port 6 of the water server 1, a trunk 3C which holds water, and a shoulder 3B connecting the neck 3A to the trunk 3C. The bottle 3 has sufficient flexibility such that as drinking water in the bottle decreases, the bottle gradually shrinks. The water bottle 3 can hold a maximum of about 12 liters of water. The water bottle 3 can be formed e.g. by blow molding of polyethylene terephthalate (PET) resin. A cap 7 is mounted on the neck 3A of the water bottle 3 to close the opening of the bottle at the distal end of the neck 3A.

The bottle insertion port 6 is provided on the top surface of the housing 2. An annular seat surface 8 is further formed on the top surface of housing 2 to surround the bottle insertion port 6. The seat surface 8 is tapered so as to gradually lower toward the bottle insertion port 6 so that the seat surface 8 can support the shoulder 3B of the water bottle 3. A tubular support frame 9 is mounted on top of the housing 2 to surround the trunk 3C of the water bottle 3, thereby keeping the flexible water bottle 3 in a stable position.

A water passing rod 11 is provided in the bottle insertion port 6, which is provided on top of the housing 2. The water passing rod 11 is configured to be water-tightly inserted through a water passage hole 10 formed in the cap 7 of the water bottle 3 when the water bottle 3 is inserted into the bottle insertion port 6. The water passing rod 11 is connected to a water introducing pipe 12 through which drinking water can be introduced into the cold water tank 4. Thus, drinking water in the water bottle 3 flows into the cold water tank 4 through the water passing rod 11 and the water introducing pipe 12.

The water introducing pipe 12 carries a float valve 13 at its end at the cold water tank 4. The float valve 13 is adapted to be opened and closed according to the vertical position of a float 14 on the surface of the drinking water in the cold water tank 4, thereby keeping constant the water level in the cold water tank 4. Specifically, as soon as the water level and thus the float 14 falls below a predetermined level, the float valve 13 is adapted to open, allowing drinking water to be introduced into the cold water tank 4. When, as a result, the level of water in the cold water tank 4 and thus the float 14 rise to the predetermined level, the float valve 13 is adapted to be closed, thereby stopping the flow of drinking water into the cold water tank.

A cooling device 15 is mounted to the cold water tank 4 for cooling the drinking water in the cold water tank 4 and keeping it at a low temperature (about 5°C). The capacity of the cold water tank 4 is smaller than that of the water bottle 3, and is about 2 to 4 liters. A cold water discharge pipe 16 is connected to a lower portion of the cold water tank 4 through which drinking water in the cold water tank 4 is discharged to outside. The cold water discharge pipe 16 is provided with a cold water cock 17 which can be operated from outside the housing 2. By opening the cold water cock 17, cold drinking water is discharged from the cold water tank 4.

A heating device 18 is mounted to the hot water tank 5 to keep the drinking water in the hot water tank 5 at a high temperature (about 90°C). The capacity of the hot water tank 5 is about 1 to 2 liters. A hot water discharge pipe 19 is connected to an upper portion of the hot water tank 5 through which drinking water in the hot water tank 5 can be discharged to outside. The hot water discharge pipe 19 is provided with a hot water cock 20 which can be operated from outside the housing 2. By opening the hot water cock 20, hot drinking water is discharged from the hot water tank 5.

The cold water tank 4 and the hot water tank 5 are connected together through a tank connecting pipe 21 such that as drinking water is discharged from the hot water tank 5, drinking water in the cold water tank 4 flows into the hot water tank 5 through the tank connecting pipe 21. In order to prevent drinking water in the cold water tank 4 that has been cooled to a low temperature by the cooling device 15 of the cold water tank 4 from flowing into the hot water tank 5 through the tank connecting pipe 21, the tank connecting pipe 21 has its top opening located in the cold water tank 4 at a level higher than the cooling device 15. In order to prevent drinking water in the hot water tank 5 that has been heated to a high temperature by the heating device 18 of the hot water tank 5 from flowing into the cold water tank 4 through the tank connecting pipe 21, the tank connecting pipe 21 has its bottom opening located in the hot water tank 5 at a level lower than the heating device 18.

When cold water is discharged from the cold water tank 4, the water level in the cold water tank 4 temporarily falls. When hot water is discharged from the hot water tank 5 too, the water level in the cold water tank 4 temporarily falls because the drinking water in the cold water tank 4 flows into the hot water tank 5 through the tank connecting pipe 21. When the water bottle 3 set in the water server 1 becomes empty too, the water level in the cold water tank 4 gradually falls as the water remaining in the cold water tank 4 decreases.

In order to introduce air into the cold water tank when the water level in the cold water tank 4 falls for the above reasons, an air introducing pipe 22 is connected to an upper portion of the cold water tank 4 through which air can be introduced into the cold water tank 4. By introducing air into the cold water tank 4 through the air introducing pipe 22 in an amount corresponding to the fall of the water level in the cold water tank 4, the interior of the cold water tank 4 is kept at the atmospheric pressure. An air sterilizing chamber 23 is connected to the air introducing pipe 22. The air sterilizing chamber 23 prevents entry of bacteria and microbes into the cold water tank 4 together with air by sterilizing air using ozone.

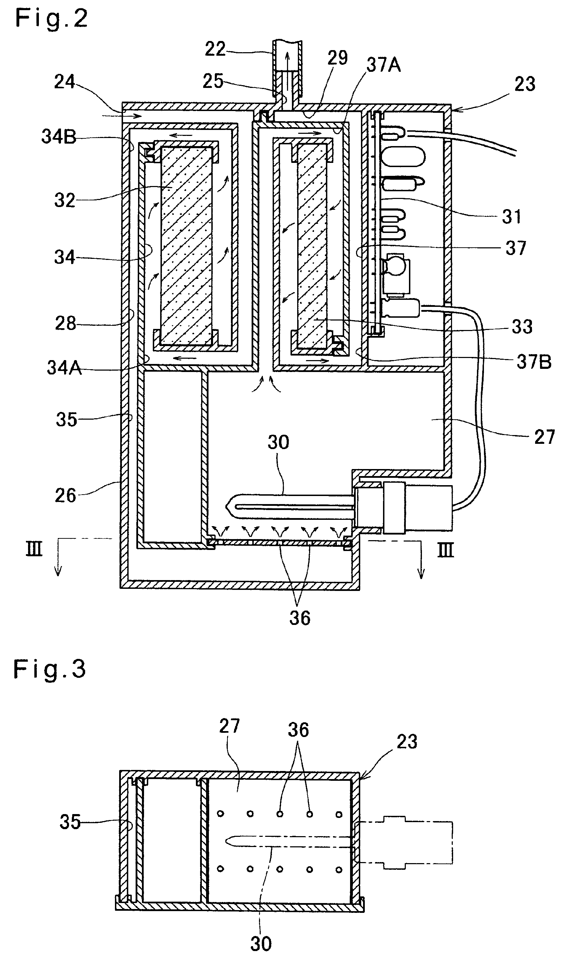

As shown in Fig. 2, the air sterilizing chamber 23 includes a case 26 having an air inlet port 24 and an air outlet port 25. The case 26 defines an air compartment 27, an air inlet passage 28 through which the air compartment 27 communicates with the air inlet port 24, and an air outlet passage 29 through which the air compartment 27 communicates with the air outlet port 25.

The case 26 is a vertically elongated hollow box. The air inlet port 24 is formed in an upper portion of the side wall of the case 26 to open to outside the case 26. The air outlet port 25 is formed in the top surface of the case 26 and is connected to the air introducing pipe 22. The air compartment 27, the air inlet passage 28 and the air outlet passage 29 are defined in the case 26 by partitioning walls. The air inlet port 24 may be formed in the top surface of the case 26. But by forming the air inlet port 24 in the side wall of the case 26, it is possible to more effectively prevent entry of foreign matter such as water and dust into the air inlet port 24.

An ozone generator 30 is provided in the air compartment 27 which converts air in the air compartment 27 to ozone. The ozone generator 30 may e.g. be a low-pressure mercury lamp, which generates ozone by UV irradiation of oxygen in the air, or a silent discharge device, which converts oxygen between an opposed pair of electrodes to ozone by applying AC voltage between the electrodes. A control board 31 is mounted in the case 26 which controls the ozone generator 30. In particular, the control board 31 intermittently activates the ozone generator 30 to keep the ozone concentration in the air compartment 27 within a predetermined range. Since ozone is larger in specific gravity than air (about 1.8 times that of air), ozone generated by the ozone generator 30 collects and stays at the lower portion of the air compartment 27 due to the difference in specific gravity.

In order to prevent ozone in the case 26 from being released into the outside environment, an inlet-side ozone decomposing filter 32 is provided in the air inlet passage 28. The inlet-side ozone decomposing filter 32 decomposes ozone that passes through the inlet side ozone decomposing filter 32 into oxygen. This filter may be an activated charcoal filter, or a filter including an ozone-decomposing catalyst (such as manganese dioxide) carried by a honeycomb aluminum substrate. An outlet-side ozone decomposing filter 33, which is identical or similar in structure to the inlet-side ozone decomposing filter 32, is provided in the air outlet passage 29.

The inlet-side ozone decomposing filter 32 is located at a higher level than the air compartment 27. The air inlet passage 28 includes a rectangular spiral path 34 enclosing the inlet-side ozone decomposing filter 32, and a connecting path 35 extending along the side wall and the bottom wall of the case 26 from the spiral path 34 to the lower portion of the air compartment 27. The spiral path 34 comprises a first portion 34A through which air flows to the inlet-side ozone-decomposing filter 32 in one of the clockwise and counterclockwise directions (clockwise direction in Fig. 2), and a second portion 34B through which air flows from the inlet-side ozone-decomposing filter 32 in the other of the clockwise and counterclockwise directions (counterclockwise direction in Fig. 2). Thus, the first portion 34A has its upstream end connected to the air inlet port 24, and the second portion 34B has its downstream end connected to the connecting path 35. The portion of the air inlet passage 28 between the air compartment 27 and the inlet-side ozone-decomposing filter 32 partially extends above the inlet-side ozone-decomposing filter 32. (In the embodiment, the second portion 34B, i.e. the portion of the spiral path 34 through which air flows from the inlet-side ozone-decomposing filter 32 partially extends above the inlet-side ozone-decomposing filter 32.

As shown in Figs. 2 and 3, a plurality of minute air holes 36 are formed in the bottom surface of the air compartment 27 through which the air compartment 27 communicates with the air inlet passage 28. Air flowing through the air holes 36 into the air compartment 27 rises in the air compartment 27 while being brought into contact with ozone in the air compartment 27. Air in the air compartment 27 is thus cleaned by contact with ozone. The minute air holes 36 serve to increase the surface area of the air flowing into the air compartment 27, thus increasing the contact area between the air and ozone. After contacting ozone, the air in the air compartment 27 flows out of the air compartment 27 through the air outlet passage 29, which is connected to the top surface of the air compartment 27.

As shown in Fig. 2, the outlet-side ozone decomposing filter 33 is located at a higher level than the air compartment 27. The air outlet passage 29 includes a rectangular spiral path 37 enclosing the outlet-side ozone decomposing filter 33. The spiral path 37 comprises a first portion 37A through which air flows to the outlet-side ozone-decomposing filter 33 in one of the clockwise and counterclockwise directions (clockwise direction in Fig. 2), and a second portion 37B through which air flows from the outlet-side ozone-decomposing filter 33 in the other of the clockwise and counterclockwise directions (counterclockwise direction in Fig. 2). Thus, the first portion 37A has its upstream end connected to the air compartment 27, and the second portion 37B has its downstream end connected to the air outlet port 25. The portion of the air outlet passage 29 between the air compartment 27 and the outlet-side ozone-decomposing filter 33 partially extends above the outlet-side ozone-decomposing filter 33. (In the embodiment, the first portion 37A, i.e. the portion of the spiral path 37 through which air flows from the outlet-side ozone-decomposing filter 33 partially extends above the outlet-side ozone decomposing filter 33.

The case 26 is made of an ozone-resistant resin to prevent the case 26 from being corroded by ozone. Ozone-resistant resins include fluororesins such as tetrafluoroethylene-perfluoroalkylvinylether copolymers (PFA resins) and tetrafluoroethylene-hexafluoropropylene copolymers (FEP resins).

With this water server 1, because the inlet-side ozone decomposing filter 32 and the outlet-side ozone decomposing filter 33 of the air sterilizing chamber 23 are both located at a higher level than the air compartment 27, and because ozone generated by the ozone generator 30 in the air compartment 27 flows downward in the case 26 due to a difference in specific gravity between ozone and air, the ozone is not brought into contact with either of the inlet-side ozone decomposing filter 32 and the outlet-side ozone decomposing filter 33. Ozone generated by the ozone generator 30 thus stays practically entirely in the case 26, making it possible to efficiently increase the ozone concentration in the case 26. This in turn makes it possible to effectively sterilize air passing through the case 26 and thus brought into contact with drinking water in the cold water tank 4.

Further, since the portion of the air inlet passage 28 between the air compartment 27 and the inlet-side ozone-decomposing filter 32 (in particular, the second portion 34B of the spiral path 34, through which air flows from the inlet-side ozone-decomposing filter32) partially extends above the inlet-side ozone-decomposing filter 32, ozone in the air compartment 27, which is larger in specific gravity than air, is even less likely to reach the inlet-side ozone-decomposing filter 32. Similarly, since the portion of the air outlet passage 29 between the air compartment 27 and the outlet-side ozone-decomposing filter 33 (in particular, the first portion 37A of the spiral path 37, through which air flows from the outlet-side ozone-decomposing filter 33) partially extends above the outlet-side ozone decomposing filter 33, ozone in the air compartment 27, which is larger in specific gravity than air, is less likely to reach the outlet-side ozone-decomposing filter 33 either. This makes it possible to prevent ozone in the air compartment 27 from being brought into contact with either of the ozone-decomposing filters 32 and 33 while air inside and outside of the case 26 is not moving (e.g. while no drinking water is being dispensed from the water server 1). Thus, this arrangement also serves to efficiently increase the ozone concentration in the case 26.

The rectangular spiral paths 34 and 37 of the air inlet passage 28 and the air outlet passage 29, in which the inlet-side and outlet-side ozone-decomposing filters 32 and 33 are enclosed, serve to increase the total lengths of the air inlet passage 28 and the air outlet passage 29, provided the interior of the case 26 remains unchanged. This arrangement thus further effectively prevents ozone in the air compartment 27 from reaching, and being decomposed by, either of the inlet-side ozone-decomposing filter 32 and the outlet-side ozone-decomposing filter 33.

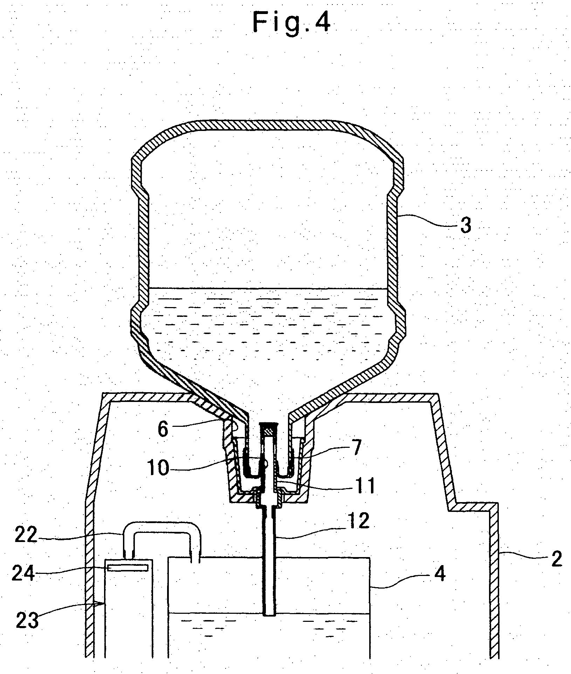

In the above-described embodiment, the water server 1 is used which is of the type that requires a water bottle 3 which shrinks as the drinking water in the bottle decreases. But as shown in Fig. 4, the present invention is applicable to a water server of the type that uses a water bottle 3 which is so hard that its shape remains unchanged as the drinking water in the bottle decreases.

In the water server of Fig. 4, no float valve 13 as used in the above embodiment is provided at the end of the water introducing pipe 12 at the cold water tank 4, so that this end of the water introducing pipe 12 is always open in the cold water tank 4. In this arrangement, the water level in the cold water tank 4 is maintained at a constant level due to the balance between the pressure in the water bottle 3 and the atmospheric pressure, which acts on the surface of the water in the cold water tank 4. In particular, as soon as the water level in the cold water tank 4 falls below the end of the water introducing pipe 12 at the cold water tank 4, drinking water in the bottle 3 flows through the water introducing pipe 12 into the cold water tank 4, while simultaneously, air in the cold water tank 4 rises through the water introducing pipe 12 and is introduced into the water bottle 3. When, as a result, the water level in the cold water tank 4 rises and reaches the end of the water introducing pipe 12 at the cold water tank 4, drinking water stops flowing through the water introducing pipe 12 into the cold water tank 4 because the pressure in the water bottle 3 balances with the atmospheric pressure, which acts on the surface of the water in the cold water tank 4.

In a further alternative arrangement, the water server includes an air introducing pipe branched from the water passing rod 11, with the air sterilizing chamber 23 connected to this air introducing pipe. With this arrangement too, it is possible to sterilize air to be brought into contact with the drinking water in the water bottle 3, thereby effectively preventing bacteria and microbes from mixing into the water bottle 3.

### DESCRIPTION OF THE NUMERALS

1. Water server
4. Cold water tank
16. Cold water discharge pipe
22. Air introducing pipe
23. Air sterilizing chamber
24. Air inlet port
25. Air outlet port
26. Case
27. Air compartment
28. Air inlet passage
29. Air outlet passage
30. Ozone generator
32. Inlet-side ozone-decomposing filter
33. Outlet-side ozone-decomposing filter
34. Spiral path
37. Spiral path

## Claims

1. A water server comprising a water tank (4) which can hold drinking water, a water discharge pipe (16) through which drinking water in the water tank (4) can be discharged, an air introducing pipe (22) through which air can be introduced into the water tank (4) as a water level in the water tank (4) falls, and an air sterilizing chamber (23) connected to the air introducing pipe (22) and configured to sterilize air in the air introducing pipe (22), wherein
the air sterilizing chamber (23) comprises:
a case (26) having an air inlet port (24) and an air outlet port (25), and defining in the case (26) an air compartment (27), an air inlet passage (28) through which the air compartment (27) communicate with the air inlet port (24), and an air outlet passage (29) through which the air compartment (27) communicates with the air outlet port (25);
an ozone generator (30) mounted in the air compartment (27) and capable of converting oxygen in the air compartment (27) to ozone;
and
an outlet-side ozone-decomposing filter (33) having activated charcoal mounted in the air outlet passage (29) at a higher level than the air compartment (27), **characterized by** an inlet-side ozone-decomposing filter (32) having activated charcoal mounted in the air inlet passage (28) at a higher level than the air compartment (27).

2. The water server of claim 1, wherein the air inlet passage (28) includes a portion (34B) extending between the air compartment (27) and the inlet-side ozone-decomposing filter (32) and partially extending above the inlet-side ozone-decomposing filter (32), and wherein the air outlet passage (29) includes a portion (37A) extending between the air compartment (27) and the outlet-side ozone-decomposing filter (33) and partially extending above the outlet-side ozone-decomposing filter (33).

3. The water server of claim 1 or 2, wherein the air inlet passage (28) includes a rectangular spiral path (34) enclosing the inlet-side ozone-decomposing filter (32), and wherein the air outlet passage (29) includes a rectangular spiral path (37) enclosing the outlet-side ozone-decomposing filter (33).

## Patentansprüche

1. Ein Wasser-Spender, der einen Wasser-Tank (4), welcher Trinkwasser halten kann, eine Wasser-Abgabeleitung (16), durch welche Trinkwasser in dem Wasser-Tank (4) abgegeben werden kann, eine Luft-Einführungsleitung (22), durch welche Luft in den Wasser-Tank (4) eingeführt werden kann, wenn ein Wasser-Niveau in dem Wasser-Tank (4) fällt, und eine Luft-Sterilisierungskammer (23), verbunden mit der Luft-Einführungsleitung (22) und konfiguriert, um Luft in der Luft-Einführungsleitung (22) zu sterilisieren, umfasst, wobei
die Luft-Sterilisierungskammer (23) umfasst:
ein Gehäuse (26), das einen Luft-Einlass-Anschluss (24) und einen Luft-Auslass-Anschluss (25) hat, und in dem Gehäuse (26) eine Luft-Abteilung (27), einen Luft-Einlass-Durchgang (28), durch den die Luft-Abteilung (27) mit dem Luft-Einlass-Anschluss (24) kommuniziert, und einen Luft-Auslass-Durchgang (29), durch den die Luft-Abteilung (27) mit dem Luft-Auslass-Anschluss (25) kommuniziert, definiert;
einen Ozon-Generator (30), montiert in der Luft-Abteilung (27) und in der Lage, Sauerstoff in der Luft-Abteilung (27) in Ozon umzuwandeln;
und einen Auslass-Seiten-Ozon-Aufspaltungs-Filter (33), der Aktivkohle hat, montiert in dem Luft-Auslass-Durchgang (29) in einem höheren Niveau als die Luft-Abteilung (27), **gekennzeichnet durch** einen Einlass- Seiten-Ozon-Aufspaltungs-Filter (32), der Aktivkohle hat, montiert in dem Luft-Einlass-Durchgang (28) in einem höheren Niveau als die Luft-Abteilung (27).

2. Der Wasser-Spender von Anspruch 1, wobei der Luft-Einlass-Durchgang (28) einen Abschnitt (34B) beinhaltet, der sich zwischen der Luft-Abteilung (27) und dem Einlass-Seiten-Ozon-Aufspaltungs-Filter (32) erstreckt und sich teilweise oberhalb des Einlass-Seiten-Ozon-Aufspaltungs-Filter (32) erstreckt, und wobei der Luft-Auslass-Durchgang (29) einen Abschnitt (37A) beinhaltet, der sich zwischen der Luft-Abteilung (27) und dem Auslass-Seiten-Ozon-Aufspaltungs-Filter (33) erstreckt und sich teilweise oberhalb des Auslass-Seiten-Ozon-Aufspaltungs-Filter (33) erstreckt.

3. Der Wasser-Spender von Anspruch 1 oder 2, wobei der Luft-Einlass-Durchgang (28) einen rechtwinkligen Spiralpfad (34) beinhaltet, der den Einlass-Seiten-Ozon-Aufspaltungs-Filter (32) einschließt, und wobei der Luft-Auslass-Durchgang (29) einen rechtwinkligen Spiralpfad (37) beinhaltet, der den Auslass-Seiten-Ozon-Aufspaltungs-Filter (33) einschließt.

## Revendications

1. Distributeur d'eau comprenant un réservoir d'eau (4) qui peut contenir de l'eau potable, un tuyau d'évacuation d'eau (16) au travers duquel peut être évacuée l'eau potable se trouvant dans le réservoir d'eau (4), un tuyau d'introduction d'air (22) au travers duquel de l'air peut être introduit dans le réservoir d'eau (4), ainsi qu'un compartiment de stérilisation d'air (23) relié au tuyau d'introduction d'air (22) et configuré pour stériliser l'air dans le tuyau d'introduction d'air (22), dans lequel
le compartiment de stérilisation d'air (23) comprend :
une enveloppe (26) possédant un orifice d'entrée d'air (24) et un orifice de sortie d'air (25), et définissant dans l'enveloppe (26) un compartiment d'air (27), un passage d'entrée d'air (28) au travers duquel le compartiment d'air (27) communique avec l'orifice d'entrée d'air (24) et un passage de sortie d'air (29) au travers duquel le compartiment d'air (27) communique avec l'orifice de sortie d'air (25),
un générateur d'ozone (30) monté dans le compartiment d'air (27) et pouvant convertir l'oxygène en ozone dans le compartiment d'air (27),
et
un filtre de décomposition d'ozone (33) du côté orifice de sortie comportant du charbon actif monté dans le passage de sortie d'air (29) à un niveau supérieur à celui du compartiment d'air (27), **caractérisé par** un filtre de décomposition d'ozone (32) du côté orifice d'entrée comportant du charbon actif monté dans le passage d'entrée d'air (28) à un niveau supérieur à celui du compartiment d'air (27).

2. Distributeur d'eau selon la revendication 1, dans lequel le passage d'entrée d'air (28) inclut une partie (34B) s'étendant entre le compartiment d'air (27) et le filtre de décomposition d'ozone (32) du côté orifice d'entrée, et s'étendant partiellement au-dessus du filtre de décomposition d'ozone (32) du côté orifice d'entrée, et dans lequel le passage de sortie d'air (29) inclut une partie (37A) s'étendant entre le compartiment d'air (27) et le filtre de décomposition d'ozone (33) du côté orifice de sortie, et s'étendant partiellement au-dessus du filtre de décomposition d'ozone (33) du côté orifice de sortie.

3. Distributeur d'eau selon la revendication 1 ou la revendication 2, dans lequel le passage d'entrée d'air (28) inclut un chemin en spirale (34) rectangulaire renfermant le filtre de décomposition d'ozone (32) du côté orifice d'entrée, et
dans lequel le passage de sortie d'air (29) inclut un chemin en spirale (37) rectangulaire renfermant le filtre de décomposition d'ozone (33) du côté orifice de sortie.
